# EUROPEAN PATENT SPECIFICATION

(11) **EP 1 208 840 B1**
(45) Date of publication and mention of the grant of the patent: **24.01.2007**
(21) Application number: 01309983.3
(22) Date of filing: 28.11.2001
(51) Int. Cl.: A61K 31/54, A61K 31/505, A61P 35/00

(54) **Combination of fluorouracil and a methylol transfer agent for the treatment of tumor metastases and cancer**
Zusammensetzung zur Behandlung von Tumoren oder Krebs, welche Fluorouracil und ein Methylol Übertragungsmittel enthält
Composition pour le traitement des métastases ou du cancer contenant du fluorouracil et un agent de transfert de métylol

(30) Priority: 28.11.2000 US 253138 P
(43) Date of publication of application: 29.05.2002
(73) Proprietor: Ed Geistlich Söhne AG Für Chemische Industrie, 6110 Wolhusen (CH)
(72) Inventor: Redmond, Paul H., Wilton, Cork (IE); Pfirrmann, Rolf W., 6006 Lucerne (CH)
(74) Representative: Marsden, John Christopher

(56) References cited:
- EP-A- 1 066 830
- WO-A-92/00743
- HANSEN L.A. ET AL: "Altretamine." DICP, ANNALS OF PHARMACOTHERAPY, (1991) 25/2 (146-152). , XP008013915
- PARFITT K (ED): "Martindale, the complete drug reference, 32nd ed" , MARTINDALE: THE COMPLETE DRUG REFERENCE. (FORMERLY MARTINDALE THE EXTRA PHARMACOPOEIA), LONDON: PHARMACEUTICAL PRESS, GB, PAGE(S) 534-537 XP002231711 ISBN: 0-85369-429-X * page 536, column 1, paragraph 6 *

## Description

The present invention relates to the field of treating tumor metastases and cancer.

5-Fluorouracil (5-FU) is an anti-neoplastic drug with clinical activity in a variety of tumors, such as cancers of the colon and rectum, head and neck, liver, breast, and pancreas. One problem with 5-FU is its extreme toxicity. Since 5-FU targets rapidly dividing cells, the primary toxic side effects are on bone marrow, intestinal mucosa and oral mucosa. Thus, leukocyte and platelet count decreases substantially after administration. Other side effects include stomatitis, diarrhea, nausea and vomiting. Neurological side effects include somnolence and ataxia. Other side effects include chest pain, myocardial necrosis and ischemia. Inflammatory reactions such as acute and chronic conjunctivitis leading to tear duct stenosis and ectropion also occur.

Monotherapy with 5-FU only results in tumor remission in about 20-25% of patients, and the average remission time is only about 6-8 months.

Although combination chemotherapy with 5-FU and other anti-neoplastic agents has been proposed, typically no substantive additional benefit is provided by the other anti-neoplastic agents over treatment with 5-FU alone.

Thus, there remains a significant need in the art for new and improved cancer treatment therapies.

In accordance with the present invention, tumor growth and metastasis is inhibited in a cancer patient by administering to said patient a combination therapy comprising effective amounts of 5-FU and a methylol transfer agent selected from taurolidine and taurultam and mixtures there of.

It has surprisingly been found that methylol transfer agents selected from taurolidine and taurultam substantially enhance or augment the anti-neoplastic effects of 5-FU in a combination therapy for inhibiting tumor metastases and treating cancer in patients. Those methylol transfer agents also substantially reduce the toxic side effects of 5-FU.

Methylol transfer agents include methylol-containing compounds such as taurolidine and taurultam. The compounds taurolidine and taurultam are disclosed in U.S. Patent No. 5,210,083. The methylol transfer agents for utilization in accordance with the present invention are taurolidine, taurultam, and mixtures thereof.

The invention involves treatment of cancers selected from the group consisting of colo-rectal cancer, as well as inhibition of tumor metastases thereof. A particularly preferred embodiment of the invention is the treatment of colo-rectal cancer.

Effective daily dosage amounts of 5-FU may be in the range of 0.1-1,000 mg per pharmaceutical dosage unit. Effective dosage amounts of 5-FU also may be in the range of 100-5,000 mg/m² body surface area, preferably 200-1,000 mg/m² body surface area, more preferably 500-600 mg/m² body surface area. 5-FU typically is provided in 250 mg or 500 mg ampules for injection, or 250 mg capsules for oral administration.

Effective dosage amounts of a methylol transfer agent in accordance with the present invention may comprise pharmaceutical dosage units within the range of 0.1-1,000 mg/kg. Preferred dosages may be in the range of 10-20 grams taurolidine, taurultam or a mixture thereof, per administration.

Pharmaceutical dosage units of the combined therapy of the present invention may be administered by any suitable route, which include oral, topical or peritoneal administration, e.g. subcutaneously, intraperitoneally, intramuscularly, or intravenously, e.g. by infusion or injection.

In preferred embodiments, 250 ml of taurolidine 2% solution is administered by intravenous infusion 1-6 times per day, more preferably 2-4 times per day, during a treatment period, concurrently or sequentially with administration of 5-FU at a preferred dosage within the range of 500-600 mg/m² body surface area. In accordance with one embodiment, 5-FU is administered by bolus intravenous injection at a dosage of 500 mg/m² body surface area, 1-3 days per week for a total of three weeks during a treatment period including administration of taurolidine and/or taurultam. In an alternative embodiment, a 600 mg/m² intravenous bolus injection is administered 1-2 times per week during a three week treatment period, along with administration of taurolidine and/or taurultam as indicated above.

In contrast with other anti-neoplastic agents, methylol transfer agents such as taurolidine and taurultam surprisingly and substantially enhance or augment the anti-neoplastic effects of 5-FU, and substantially reduce the extreme toxic side effects of 5-FU, e.g. myelosuppression, stomatitis, diarrhea, nausea and vomiting, without reducing its efficacy. Accordingly, with a combination therapy of 5-FU and a methylol transfer agent such as taurolidine and/or taurultam, the amount of 5-FU can be reduced to achieve the same activity as larger dosages of 5-FU alone, while encountering fewer toxic side effects. Alternatively, combination therapy in accordance with the present invention can be utilized with the same 5-FU dosage levels as monotherapy with 5-FU, while achieving enhanced anti-neoplastic results along with fewer side effects.

The invention is further illustrated by the following example.

### Example 1

The human colo-rectal cell lines SW 480 (primary), SW 620 (metastatic) and W 707 (metastatic) were incubated with the following: culture medium (control), taurolidine at 5, 10, 25, 50 and 100 µg/ml doses, and 5-Fluorouracil (5-FU) at 5, 10, 25, 50 and 100 µM doses. 5-FU was tested alone, and together with taurolidine. Cell proliferation, apoptosis and cell cycle were assessed.

There was a significant decrease in tumor cell proliferation at 24 hours. There was no significant increase in taurolidine-induced apoptosis and taurolidine did not alter the phases of the cell cycle. There was an increase in LDH release (p = 0.0011), which correlated with inhibited tumor proliferation. Taurolidine was found to augment the effects of given doses of 5-FU (p = 0.0001).

## Claims

1. Use of 5-fluorouracil and a methylol transfer agent selected from tauroliline, taurultam and mixtures thereof in the manufacture of medicaments for simultaneous, separate or sequential use in inhibiting colo-rectal cancer or tumour metastases thereof in a cancer patient.

2. Use as claimed in claim 1 wherein said 5-fluorouracil and said methylol transfer agent are each presented as separate pharamaceutical dosage units.

## Patentansprüche

1. Verwendung von 5-Fluoruracil und eines Methylol-Übertragungsmittels, das aus Taurolidin, Taurultam und Gemischen davon ausgewählt ist, zur Herstellung von Arzneimitteln zur gleichzeitigen, getrennten oder sequenziellen Verwendung zur Hemmung von kolorektalem Krebs oder Tumormetastasen davon bei einem Krebspatienten.

2. Verwendung wie in Anspruch 1 beansprucht, bei der das 5-Fluoruracil und das Methylol-Übertragungsmittel jeweils als getrennte pharmazeutische Dosierungseinheiten vorliegen.

## Revendications

1. Utilisation de 5-fluorouracile et d'un agent de transfert de méthylol choisi parmi la taurolidine, le taurultam et leurs mélanges, pour la fabrication de médicaments pour une utilisation simultanée, séparée ou séquentielle pour inhiber le cancer colo-rectal ou les métastases tumorales de ce cancer chez un patient cancéreux.

2. Utilisation selon la revendication 1, dans laquelle ledit 5-fluorouracile et ledit agent de transfert de méthylol sont présentés chacun en formes de dosage unitaires pharmaceutiques distinctes.
